# EUROPEAN PATENT APPLICATION

(11) **EP 1 886 671 A1**
(43) Date of publication of application: **13.02.2008**
(21) Application number: 06016692.3
(22) Date of filing: 10.08.2006
(51) Int. Cl.: A61K 9/20, A61K 47/02, A61K 47/36

(54) **Starch silica co-precipitate, method for preparing the same and use thereof**

(71) Applicant: The Jordanian Pharmaceutical Manufacturing Co., 11710 Naor (JO)
(72) Inventor: Badwan, Adnan, 11185 Amman (JO); Al-Remawi, Mayyas, 13713 Russiefa (JO); Rashid, Iyad Said, 11151 Amman (JO)
(74) Representative: Winkler, Andreas Fritz Ernst

(57) **Abstract**

The invention is related to a co-precipitate comprising at least one hydrophilic polymeric compound selected from the group consisting of hydrophilic starch or its derivatives, and at least one water-absorbable compound selected from the group consisting of silicon dioxide, derivatives thereof, or any mixtures thereof, a method of preparing the same and use thereof.

## Description

### Field of Invention

The present invention relates to a co-precipitate, a method for preparing the same as well as an use of the co-precipitate as a filler and/or a disintegrant for solid pharmaceutical dosage forms.

### Background of the Invention

Solid pharmaceutical dosage forms consist in most cases of powder mixtures of active material and pharmaceutical excipients.

### The excipients can be classified into the following:

Excipients that are necessary for maintaining the physical structure of the solid dosage form before the patient intake including diluents, binders, and lubricants.

Excipients which help to give additional desirable physical characteristics to the finished tablet include colors, and, in the case of chewable tablets, flavors, sweetening agents, and coating materials.

Excipients playing a role during tablet formation are called filler/binder or excipient needed for bond formation. It is a substance added to facilitate formation of a hard tablet upon compaction of the powder formula especially with poorly compactable active materials. Fillers usually have powerful flow and compaction properties such as microcrystalline cellulose.

Excipients that play a role after the ingestion of the solid dosage form are called disintegrants. It is a substance, or a mixture of substances, added to a tablet to facilitate its breakup or disintegration after administration. The active ingredient must be released from the tablet matrix as efficiently as possible to allow for a rapid dissolution. Materials serving as disintegrants have been chemically classified as starches, clays, celluloses, algins, or gums.

The most popular disintegrants are corn and potato starch which have been well-dried and powdered.

Starch consists of two polysaccharides, amylose and amylopectin. Starch is well known to be used as a glidant; tablet binder; tablet and capsule diluent; tablet and capsule disintegrant.

Starch suffers from bad flowability, incompressibility, and poor disintegration power. However, starch is considered as a cheap excipient.

Due to its low price, many attempts have been proposed to solve these problems. For example, to solve the problem of poor disintegration power, starch derivatives were prepared. One of the most important and widely used is the sodium starch glycolate also known as carboxymethyl starch or primojel.

Sodium starch glycolate is widely used in oral pharmaceuticals as a disintegrant in capsule and tablet formulations. The usual concentration employed in a formulation is between 2-8% w/w, with the optimum concentration about 4% although in many cases 2% is sufficient. Disintegration occurs by rapid uptake of water followed by rapid and enormous swelling. However, this starch derivative suffers from bad flowability and relatively poor compactability.

Another starch derivative is pregelatinized starch. It is a starch that has been chemically and/or mechanically processed to rupture all or part of the starch granules and so render the starch flowable and directly compressible. However, this starch suffers from relatively lower disintegration power than sodium starch glycolate. The concentration to be used as a tablet disintegrant is in the range of 5-20% w/w.

### Summary of the Invention

It is therefore an object of the present invention to provide excipients for solid pharmaceutical dosage forms which overcome the drawbacks of the prior art, which show particularly improved disintegration power, flowability and compactability. Further, a method for preparing such excipients and uses thereof shall be provided.

The first object is achieved by a co-precipitate according to claim 1. A method for its preparation is disclosed in claim 10, whereas a use of such a co-precipitate is disclosed in claim 13. Preferred embodiments are disclosed in the sub-claims.

The invention is related to a co-precipitate comprising at least one hydrophilic polymeric compound selected from the group consisting of hydrophilic starch or its derivatives, and at least one water-absorbable compound selected from the group consisting of silicone dioxide, derivatives thereof, or any mixtures thereof.

Moreover, the invention is concerned with a method for preparing a co-precipitate of the invention by mixing a wet mass of the hydrophilic polymeric compound with a suspension or solution of the water-absorbable compound in an appropriate ratio and adjusting the process conditions in order to allow the co-precipitate to form.

Finally, the invention is related to the use of a co-precipitate according to the invention as a filler and/or disintegrant for solid pharmaceutical dosage forms.

It also provides additional feature i.e. it results in a compactable mixture that increases the tablet hardness.

Surprisingly, the novel starch silica coprecipitate improves both the disintegration power and the powder physical properties including flowability and compactability more than the previously mentioned starch derivatives.

In the present invention, a novel composition excipient of starch or its derivatives and silica plays the role of the disintegrant and filler at the same time.

### Detailed Description of the Invention

Starch or its derivatives or related compounds co-processed with silicon dioxide or related compounds have been found to have a superior disintegration activity. Starch is a hydrophilic polymer that is water-insoluble, but still can absorb water. This character gives starch a disintegration power. However, the substance has a poor flow and bad compression properties. The co-precipitation with silicon dioxide or related water-absorbant compounds provides the polymer with proper flow and compression properties, and increases the power of polymer for water absorption

When starch or its derivatives are co-precipitated with silicon dioxide, preferably colloidal silicon dioxide, it has been found that the resultant excipient product surprisingly provides disintegration power which is substantially improved even in comparison to normal commercially available disintegrants. Another surprising effect is the powerful improvement in flow and compaction of the new co-precipitate excipient compared to each component when taken alone.

The optimal composition contains silicon dioxide in the range of 1-90% w/w, and the most preferable concentration is about 40-60% w/w.

Starch or starch derivative, in the form of dry or wet mass is mixed with suspension or solution of silicon dioxide or related compound. The resultant co-precipitate is substantially water-insoluble. Therefore, there is no appreciable dissolution of either ingredient in the aqueous slurry. After a uniform mixture of the ingredients is obtained in a wet mass, the wet mass is dried to provide starch-silica based excipient particles.

The pH of the mixture of the compounds of the co-precipitate of the invention depends on the preparation method. The preferred pH is 4-10, typically in the range of 4 to 7.

Starch as a material for use herein, is preferably obtained from potato or wheat or rice or tapioca or corn. Particularly suitable starch for use herein is the corn starch.

Starch derivatives also can be included herein in form of cross-linked starch or starch salts including organic and inorganic salts known in the art.

Particularly suitable modified starch for use herein includes water-soluble or water-insoluble covalently bonded starch derivatives or ionically bonded derivatives known in the art.

According to the present invention the co-precipitates comprise as an essential component a water-absorbant compound, such as silicon dioxide, a derivative thereof (such as silicate) or a mixture thereof.

Silicon dioxide exists in a variety of crystalline forms and amorphous modifications, any of which are suitable for the use herein. In particular, silicon dioxide having a high surface area is preferred such as colloidal silica.

According to the present invention the articles typically comprise from 5 to 300gm⁻² more preferably from 10-250 gm⁻², most preferably from 15 to 200 gm⁻² of silica.

Optional agents such as fillers, lubricants, pH-buffering agents, coloring agents may be included to facilitate and/or improve coprecipitate formation or physical properties.

Methods of co-precipitation may include any industrial technique known such as wet or dry granulation, pH change coprecipitation, spray drying; freeze drying or simple solution mixing.

Starch silica is a superdisintegrating filler product which is mainly presented in solid form. It can be used as filler and disintegrant in immediate release solid dosage forms.

Silicated starch when compressed, using any compression machine known in the art, forms a strong compact. This compact exhibits a superdisintegration power when exposed to aqueous medium. Therefore, the novel excipient is regarded as superdisintegrating filler.

Additional features and advantages of the subject-matter of the present invention will become apparent from the following detailed description of examples with reference to the accompanying drawings, wherein
**Figure 1** is a FTIR spectra of A) starch silica co-precipitate and B) starch silica physical mixture, and
**Figure 2** shows DSC thermograms of A) starch silica co-precipitate and B) starch silica physical mixture.

### EXAMPLES

### Example 1

### Stages of starch silica superdisintegrant development

The incompressibility, bad flowability and low cost of corn starch make it a challenging option regarding the pioneering field of superdisintegrating fillers that could be of useful applications to pharmaceutical industries.

Development on the work which involved modification of corn starch was basically depending upon what kind of properties are of significant concern for industrial proposes. Three of these properties were of great interest to undertake in this piece of work. These include: Compactability, disintegration power and flowability

For example, some of these modifications have lead to the production of a highly disintegrable material but with poor compactability and flowability. This may be a good advantage when the modified type of starch is only required as an additive for tablet disintegration only but not to act as a filler. Thus, the core of this work was intensified upon modifications that could gather the three interests in one product.

As a start up to product development that could find a way for industrial application, it was important to adopt simplicity of the process in terms of operating procedures, equipment and materials used, cost, the number of components (raw materials, binders), ability to scale-up, compatibility with different drugs, etc. Three major bench-scale methods were considered for starch modification, all of them included corn starch and silica (as Aerosil 200) as the basic raw materials, and these include:
1. Granulation of starch and silica
2. Modification of corn starch
3. Coprecipitation of silica on starch

These are illustrated in examples 2-4

### Example 2

### Granulation

Corn starch was granulated with silicon dioxide (as Aerosil 200) at 1:1 mass ratio with or without using different binders. The granules were sieved and dried prior to compression using a single punch-tableting machine adjusted at 50 KN and using a 12 mm circular die. Tablet weight was fixed at 500 mg. The following table summarises the formulas that were carried out with respect to granulation. Results of physical properties are also shown in Table 1.

**Table 1: Summary of different formulations using granulation process**

| Formula # | Granulating solution | Tablet Hardness, N | Disintegration Time | Flowability |
|---|---|---|---|---|
| 1 | Deionised water | 30 | <10 seconds | Good |
| 2 | Ethanol 96% | 20 | <10 seconds | Moderate |
| 3 | 7.5% starch mucilage | 30 | <10 seconds | Good |
| 4 | 7.5% PVP K30 in water | 50 | <10 seconds | Good |
| 5 | 7.5% PVP K90 in water | 65 | <10 seconds | Good |
| 6 | 7.5% Klucel EF in Ethanol | 30 | <10 seconds | Good |
| 7 | 7.5% Klucel EF in water | 100 | <10 seconds | Good |

As shown in Table 1, it is clear that Aerosil 200 by itself provides superdisintegration properties to tablets but with poor compactability that could be only minimized through wet granulation. Hydroxypropyl cellulose (Klucel EF) provided better binding capabilities between starch and Aerosil. However, the high cost and the incompatibility of Klucel with certain drugs may make process development over superdisintegrating filler from starch an inappropriate option through wet granulation.

### Example 3

### Modification of Starch

Two processes were carried out for starch modification prior to testing the binding and disintegration powers with Aerosil 200.
1. Autocalving of starch: Corn starch had been autoclaved at 121 °C for 15 minutes, then dried up in the oven to complete dryness. The granules were crushed into fine powder through mesh #22, finally, compressed at 12 mm diameter. The following table summarizes the formulas that were carried out with respect to the autoclaved starch and Aerosil 200.

| Formula # | Process | Hardness, N | Disintegration time. | Flowability |
|---|---|---|---|---|
| 1 | Compressing the autoclaved starch as it is | 250 | >10 minutes | Good |
| 2 | Granulating the autoclaved Starch with aerosil 1:1 w/w | < 20 | <10 seconds | Good |
| 3 | Granulating the autoclaved Starch with aerosil 2:1 w/w | <20 | <10 seconds | Good |
| 4 | Granulating the autoclaved Starch with aerosil 10:1 w/w | <40 | <30 seconds | Good |

It is clear that Aerosil weakens the hardness that was achieved by autoclaving corn starch into the autoclaved form to a great extent even when using the minimum amounts of Aerosil. Autoclaving of starch as it is results in a powerful highly compressible material that can be used as filler. Nevertheless, autoclaving is not scalable, high cost process and, cannot achieve starch as superdisintegrating filler to solid dosage forms.
However, autoclaved starch may find some applications as a filler with non-disintegrating ability.
2. Heating up a solution of starch followed by coprecipitation with silica:
Corn starch was slowly added to boiling water at a concentration of 4%. Aerosil was then added to the starch solution at the ratios of 1:1 and 2:1. The suspended particulates formed were filtered out either by the addition of ethanol 96% or left to sediment under gravity over 12 hours. The filter cake was dried up and sieved into fine powder which was compressed using a 12 mm circular punch at 50 KN. Table 2 below summarizes the results obtained:

**Table 2: Summary of formulation using starch silica heating process**

| Formula# | Ratio of starch to Aerosil w/w | Hardness, N | Disintegration time | Flowability |
|---|---|---|---|---|
| 1 | 1:1 | 100 | <10 seconds | Good |
| 2 | 2:1 | 100 | 1 minute | Good |

This process provided a better quality for starch modification in terms of the physical properties needed for starch to act as disintegrating filler. However, heating is a burden to any process optimization therefore this element was left as one option and excluded in the other as would be seen in the next stage of starch development.

### Example 4:

### Coprecipitation of Starch with Aerosil 200 in Sodium Hydroxide solution

Aerosil was added to a sodium hydroxide solution (0.1-0.5M) to which corn starch was slowly added with vigorous stirring at the ratio of 1:1. The pH of the mixture was adjusted with hydrochloric acid to pH 7.0. The solid particulates were filtered out and dried up in the oven. The compressed tablets (12 mm circular) had the following physical characteristics:
Hardness: 300 N
Disintegration: <10 seconds
Flowability: Good.

This procedure gave the optimum operating conditions required to produce fast disintegrating tablets with good hardness.

It is possible for corn starch to undergo process development to work as a superdisintegrating filler by modification of the corn starch or by co-precipitation with silicone dioxide.

### Example 5:

### Comparison between the novel superdisintegrant and other starch disintegrants

Corn starch, primojel and pregelatinized starch were used as examples to be compared with the novel superdisintegrating filler. Bulk and tap densities from which polymer flowability is deduced, were calculated by measuring the weight in grams of a certain volume of the previously mentioned polymers. Compressibility and disintegration properties were carried out by compressing tablets containing the polymers using a single punch tableting machine (Manesty, UK) at a force of 50 KN and using a 12 mm circular die. Tablet weight was fixed at 500 mg. Table 3 summarizes the results.

**Table 3: Summary of physical properties of our novel starch silica coprecipitate compared to other commercially available starch disintegrants**

| Physical property | Starch* | Sodium starch glycolate | Pregelatinized starch | Starch silica coprecipitate |
|---|---|---|---|---|
| Bulk density (g/cc) | 0.516 | 0.721 | 0.580 | 0.832 |
| tap density (g/cc) | 0.677 | 0.901 | 0.751 | 0.945 |
| compressibility Index | 23.81 | 20.00 | 22.73 | 11.96 |
| Flowability | poor flow | Fair passable flow | Fair passable flow | Excellent flow |
| Hardness (N) | 35-40 | >300 | 150 | 300 |
| Disintegration time | 55-60 sec | >10 min | > 10 min | <10 sec |

| | | | | |
|---|---|---|---|---|
| * Starch compaction produced stickiness problems and tablet capping | | | | |

Starch silica coprecipitate compared to starch gives a harder tablet with higher disintegration power.

For the case of sodium starch glycloate and pregelatinized starch, which produced hard but not disintegrating tablet and its flow was fair passable compared to the novel starch silica co-precipitate. This means sodium starch glycolate could not be used as a filler in tablet formulation.

### Example 6

### Characterization of starch silica coprecipitate

To prove chemical structure similarity between starch silica coprecipitate and starch silica physical mixture IR and DSC studies were conducted.

Infrared were obtained using FTIR 480 (Jasco, Japan). Fourier transform Infra red spectrometer under room air at room temperature and KBr disk. Samples were placed in an oven at 105 °C for 3 hrs before doing any measurements to get rid of moisture. Approximately 150 mg of KBr and 5 mg of sample powder were blended with pestle and mortar for 5 min. The sample disk was prepared at a pressure of 9 tons for 21 min.

The samples were analyzed using differential scanning calorimeter DSC (heating rate 10 °C/min), with sample size 5-10 mg, and open pan DSC studies were conducted.

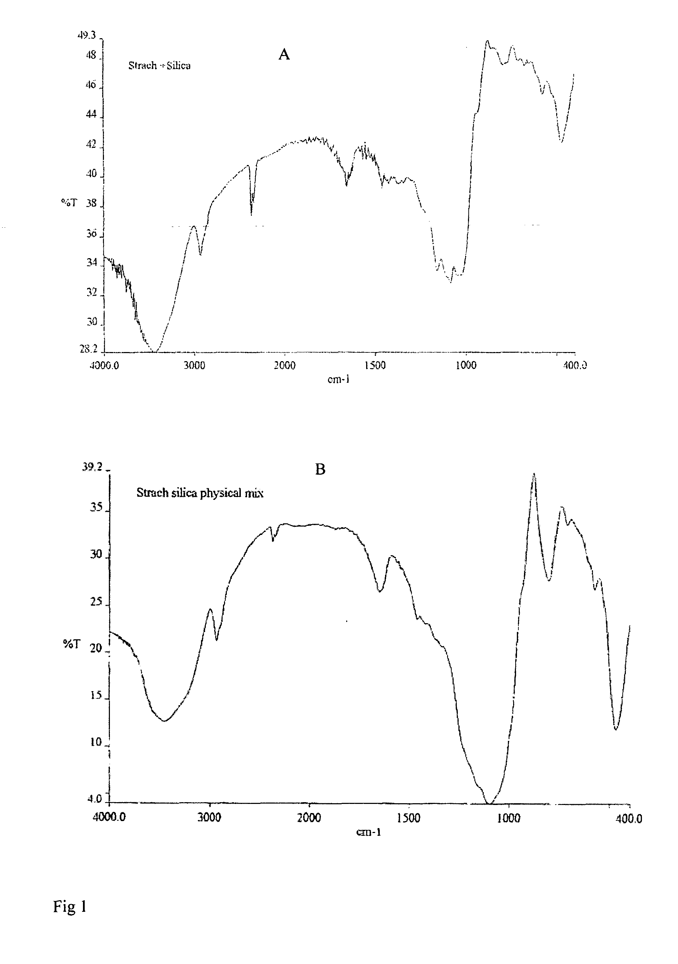

The IR spectra of the physical mixture of starch and silica showed as a similar IR spectra with a smooth IR spectra with no projections or sharpness in case of physical mix, as shown in Fig. 1. While, the coprecipitated silicated starch showed sharp peaks and the presence of shoulders with the major peaks. This indicates that the molecules were presented in an ordered crystalline state. This may be the cause for the improvement in flowability and compactability.

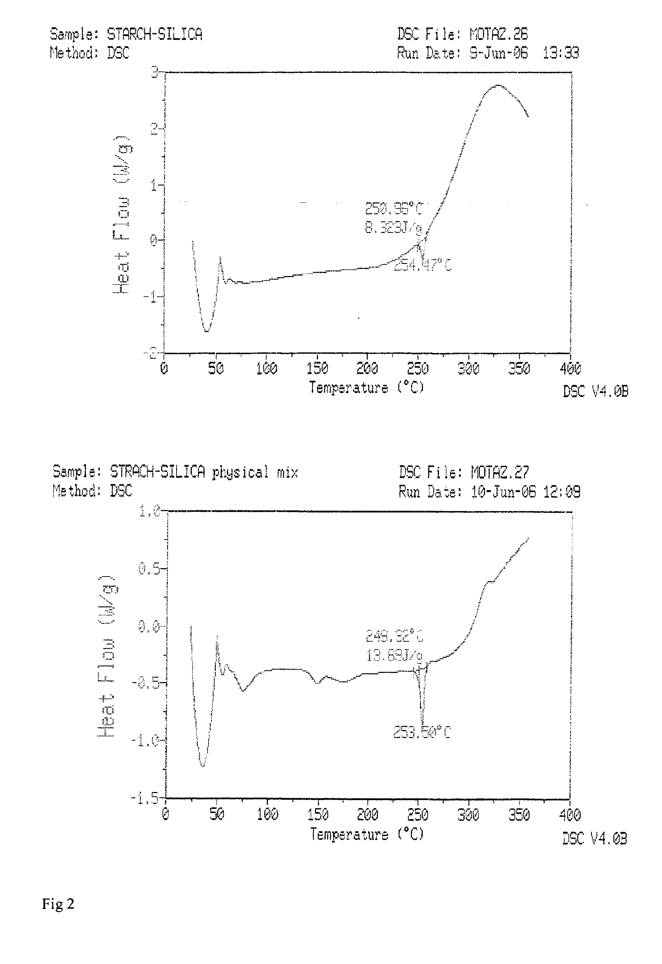

DSC thermograms illustrated in Figure 2 showed no change in the DSC scans which means similarity in chemical structure between the coprecipiate and the physical mixture.

These results indicated that the coprecipitation method resulted in production of crystalline forms of starch within the amorphous matrix. This improves its physical properties as shown in the previous example.

The features disclosed in the foregoing description, in the claims and/or in the accompanying drawings may, both separately and in any combination thereof, be material for realizing the invention in diverse forms thereof.

## Claims

1. Co-precipitate comprising at least one hydrophilic polymeric compound selected from the group consisting of hydrophilic starch or its derivatives, and at least one water-absorbable compound selected from the group consisting of silicon dioxide, derivatives thereof, or any mixtures thereof.

2. Co-precipitate according to claim 1, wherein the water-absorbable compound is selected from the group consisting of silicon dioxide and silicates.

3. Co-precipitate according to claim 2, wherein the silicon dioxide is a colloidal silicon dioxide.

4. Co-precipitate according to any of the preceding claims, wherein the hydrophilic polymeric compound is selected from the group consisting of cross-linked starch or starch salts including organic and inorganic salts.

5. Co-precipitate according to any of the preceding claims, wherein the hydrophilic polymeric compound is selected from the group consisting of water-soluble or water-insoluble, covalently bonded starch derivatives or ionically bonded derivatives.

6. Co-precipitate according to any of the preceding claims, wherein the hydrophilic polymeric compound is potato, wheat, rice, tapioca and/or corn starch, preferably corn starch.

7. Co-precipitate according to any of the preceding claims, wherein the amount of the water-absorbable compound in the co-precipitate is in the range of 1-90% w/w, preferably 40-60% w/w.

8. Co-precipitate according to any of the preceding claims, wherein the water-absorbable compound has a surface area according to BET from about 5 to about 300 m²/g, preferably 10-250 m²/g, most preferably 15 to 200 m²/g.

9. Co-precipitate according to any of the preceding claims, further comprising additional agents selected from the group consisting of fillers, lubricants, pH-buffering agents, and colouring agents.

10. Method for preparing a co-precipitate according to any of the preceding claims by mixing a wet mass of the hydrophilic polymeric compound with suspension or solution of the water-absorbable compound in an appropriate ratio and adjusting the process conditions in order to allow the co-precipitate to form.

11. Method according to claim 10, wherein the pH of the mixing solution is between 4 and 10, preferably between 4 and 7.

12. Method according to claim 10 or 11, wherein the method for co-precipitation is accomplished by wet or dry granulation, pH change co-precipitation, spray drying, freeze drying or simple solution mixing.

13. Method according to any of the claims 10 to 12, wherein the mixing is in an alkaline hydroxide solution.

14. Use of a co-precipitate according to any of the claims 1 to 10 as a filler and/or disintegrant for solid pharmaceutical dosage forms.
